# EUROPEAN PATENT APPLICATION

(11) **EP 3 081 178 A1**
(43) Date of publication of application: **19.10.2016**
(21) Application number: 14870241.8
(22) Date of filing: 30.10.2014
(51) Int. Cl.: A61B 17/70, A61B 17/88

(54) **BONE FILLER IMPLANTING SYSTEM**

(30) Priority: 12.12.2013 CN 201310671738
(71) Applicant: Ningbo Hicren Biotechnology Co. Ltd., Ningbo, Zhejiang 315336 (CN)
(72) Inventor: LV, Shiwen, Ningbo Zhejiang 315336 (CN); WANG, Yu, Ningbo Zhejiang 315336 (CN); MAO, Keya, Ningbo Zhejiang 315336 (CN); XIN, Chaohua, Ningbo Zhejiang 315336 (CN)
(74) Representative: Inchingalo, Simona
(86) International application number: PCT/CN2014/089865
(87) International publication number: WO 2015/085836

(57) **Abstract**

A bone filler implanting system comprises a bag (1) and a delivery device (2). A distal end of the bag (1) is fixedly connected to a sealing head (11); the sealing head is closed at its distal end and has a blind hole (111) at its proximal end, and the blind hole (11) has an opening towards the interior of the bag (1). The bag (1) has micro-pores (12). The delivery device (2) comprises a liner core (23), a delivery tube (21), and a sleeve tube (22). The delivery tube (21) is a rigid tube, and a distal end of the delivery tube (21) is detachably connected to a proximal end of the bag (1). The liner core (23) is slidably inserted into the delivery tube (21). A distal end part of the liner core (23) is a curved section (231), and said curved section (231) extends out of the distal end of the delivery tube (21). The distal end of the liner core (23) is inserted into the blind hole (111) of the sealing head (11) through the delivery tube (21) and the interior of the bag (1); the bag (1) wraps the curved section (231) of the liner core (23). A distal end part of the sleeve tube (22) is a flexible section (221), and a proximal end part of the sleeve tube (22) is a rigid section (222); said flexible section (221) is rigid in the radial direction. The sleeve tube (22) is sleeved on the delivery tube (21) and the bag (1). When the delivery device (2) is operated, the sleeve tube (22) slides axially towards the proximal end of the conveying tube (21), and the bag (1) is exposed.

## Description

### Cross Reference to Related Applications

This application claims the benefit of priority of Chinese Patent Application No. 201310671738.6, filed on December 12, 2013, entitled "Bone Filler Implanting System", the entire contents of which are incorporated herein by reference.

### Technical Field

The present disclosure relates to the field of medical equipment, and more particularly, to a bone filler implanting system for use in percutaneous kyphoplasty (PKP).

### Background

Osteoporotic vertebral compression fracture is a common disease harmful to the health of middle-aged and aged people. For vertebral compression fractures, traditional treatment methods are conservative mostly, including bed rest, drug analgesia, external fixation with orthosis, etc., which are easy to result in further loss of bone mass and exacerbation of osteoporosis, and thus forming a vicious circle, while the open surgery, in general, is easy to result in failures of internal fixation due to the poor health condition of the patient or insufficient fixation strength of screws.

In 1987, French doctors, Deramond et al., reported that good curative effects had been achieved for treating aggressive hemangioma of C2 vertebral body by percutaneous vertebroplasty (PVP). And hereafter, such technology is also applied in treatment of the malignant vertebral tumor and osteoporosis. The defects of such surgery include that, it is only possible to deformity-fix the injured vertebral body and alleviate the pain, but it is impossible to restore the vertebral height or correct the kyphotic deformity. Moreover, since the low-viscosity bone cement is injected into the vertebral cancellous bone directly under a relatively higher pressure, it is difficult to control the flow, and the leakage rate of the bone cement is higher, which is within the range of 30% - 67% as reported in references. By 1994, American scholars, Reiley et al., designed a new technique for correcting kyphotic deformity by means of balloon dilation based on PVP, and such technique was developed as the percutaneous kyphoplasty (PKP) that was approved by FDA in 1998 for clinical applications. Such technique includes the steps of inserting a dilatable balloon into a collapsed vertebral body by percutaneous puncture, lifting up the endplate by the dilation of the balloon, restoring the height of the vertebral body and correcting the kyphotic deformity, thereby a hollow cavity surrounded by the bone shell is formed in the vertebral body and injected with the high-viscosity bone cement under a relatively lower pressure. In order to insert the balloon into the vertebral body smoothly, a working channel has to be established primarily with a puncture needle and a working sleeve tube, then a working cavity for the un-dilated balloon is drilled with a bone drill, and finally the balloon is inserted into the vertebral body, it can be seen that the surgical procedures are complicated. Because of the structure of the vertebral body, the balloon dilation has to be operated at both sides of each vertebral body in order to maintain the biomechanical force balance of the vertebral body. What's more, in the process of the balloon dilation, original cracks of the vertebral body are often caused to be increased, and along the cracks, the bone cement leaks into the surrounding bone tissues and even leaks out of the vertebral body, causing severe complications.

Spineology Inc. has developed Vessel series of bone filler bags (Publication No: US20040073308). The bag with a hole is fed into the vertebral body, and the bone cement is injected into the bag, thereby effectively avoiding the leakage of bone cement. However, other instruments are needed to open a cavity inside the vertebral body to accommodate the bag. What's more, the puncture needs to be operated at both sides, and many steps need to be implemented in the surgical procedure, which increase the incidence of complication, prolong the duration of surgery, increase the pains of patients, and increase the economical burden on patients.

### Summary

The present disclosure aims to provide a bone filler implanting system, which can be fed to the center of the vertebral body simply by puncturing once, and which can simplify the surgery procedures, avoid the leakage of bone cement, and recover the height of the vertebral body. According to the present disclosure, a dilatable bag is fed to the center of the vertebral body through a delivery device with a curved section at its distal end. The bone filler is injected into the bag through the delivery device, thereby recovering the height of the vertebral body and avoiding the leakage of bone cement.

An objective of the present invention is realized by the following technical scheme: a bone filler implanting system comprises a bag and a delivery device; a distal end of the bag is fixedly connected to a sealing head, the sealing head is closed at its distal end and has a blind hole at its proximal end, and the blind hole has an opening towards an interior of the bag; the bag has micro-pores; the delivery device comprises a liner core, a delivery tube and a sleeve tube; the delivery tube is rigid, and a distal end of the delivery tube is detachably connected to a proximal end of the bag; the liner core is slidably inserted into the delivery tube; a distal end part of the liner core is a curved section, which extends out of the distal end of the delivery tube; a distal end of the liner core is configured to be inserted into the blind hole of the sealing head through the delivery tube and the interior of said bag; the bag is configured to wrap the curved section of the liner core; a distal end part of the sleeve tube is a flexible section, whereas a proximal end part of the sleeve tube is a rigid section, the flexible section is rigid in its radial direction; the sleeve tube is sleeved on the delivery tube and the bag; when the delivery device is operated, the sleeve tube moves axially towards a proximal end of the delivery tube, and the bag is exposed.

Further objectives of the present invention may be realized by the following technical schemes:
In one of the embodiments, a diameter of the micro-pore disposed at the proximal end part of the bag is less than the diameter of the micro-pore disposed at the distal end part of the bag; or the number of the micro-pores disposed in an intermediate part of the bag is larger than the number of the micro-pores disposed at two end parts of the bag; or the micro-pores are provided at one side of the bag; or any combination of the above.

In one of the embodiments, the bag is in a banana shape or a dumbbell shape.

In one of the embodiments, the bag is made of non-braided fiber.

In one of the embodiments, the bag is foldable in its axial direction, and is configured to wrap the distal end part of the liner core along a circumferential direction of the bag.

In one of the embodiments, an end part of the curved section is a straight section, which can be slidably inserted to the blind hole of the sealing head and finally reach a bottom of the blind hole.

In one of the embodiments, the delivery device further comprises a liner core handle fixedly connected to a proximal end of the liner core, a delivery handle fixedly connected to the proximal end of the delivery tube, and a sleeve tube handle connected to a proximal end of the sleeve tube; the delivery handle is provided with one or more guide grooves; a distal end of the delivery handle is provided with a stop block for limiting a position of the sleeve tube handle; and the sleeve tube handle is provided with one or more guide blocks engaged with the guide grooves; when the sleeve tube handle is moved, the sleeve tube moves axially together with the sleeve tube handle.

In one of the embodiments, the flexible section of the sleeve tube is cut from a metal tube, is formed of a spring, or is made of a hard medical polymer material or a combination of metal material or polymer material.

In one of the embodiments, a pressing tube is arranged between the delivery tube and the sleeve tube; a proximal end of the pressing tube is fixedly connected with a pressing tube handle; the distal end of the delivery tube or the distal end of the pressing tube is subject to deforming treatment, so that a line contact or a surface contact is formed when the distal end of the delivery tube and the distal end of the pressing tube are assembled coaxially, thereby the proximal end of the bag is fixed between the pressing tube and the delivery tube.

In one of the embodiments, the deforming treatment is flaring, closing, forming a step, fixing a bulge, or enlarging an inner cavity.

In one of the embodiments, the distal end part of the liner core is a curved section with shape memory property.

In one of the embodiments, the sealing head is made of rigid material; a distal end of the sealing head is in a sharp shape; and the distal end of the sleeve tube is sleeved on or connected to the sealing head.

As compared with the prior art, the present disclosure has the following characteristics and advantages:
1. In the prior art, before using a balloon, a channel for delivering the balloon has to be drilled with a bone drill. In view of the defect of complicated procedures, the bag of the present disclosure is provided with a hard sealing head at the distal end thereof, and furthermore, a rigid sleeve tube is sleeved on the bag, such that when delivering the bag to the vertebral body, it is unnecessary to drill a working cavity for the balloon in advance like that in the traditional PKP surgery. Owing to the protection effect of the sleeve tube on the bag, the bag can be delivered into the vertebral body directly, such that the surgical procedures are simplified and the risk of surgery is lowered, and owing to the reinforcing effect of the liner core, the procedure of inserting the bone filler implanting system into the vertebral body can be implemented more reliably and conveniently.
2. The sleeve tube of the present disclosure is rigid in the radial direction, such that it can withstand a certain pressure, and the bone tissue can be pushed away by the curved section through rotating the handle, so as to deliver the bone filler implanting system to the vertebral body more smoothly.
3. The distal end part of the liner core is a curved section, and the distal end part of the sleeve tube is a flexible section, which enable the bone filler implanting system of the present disclosure to get to the center of the vertebral body or the opposite side of the vertebral body simply by puncturing at one side, thereby avoiding the complicated puncture operation at both sides and the defect of high incidence of complications. The design of the rigid proximal end part of the sleeve tube enables that the push force can be transferred better and that the puncture can be implemented easier.
4. In a clinical surgery, once the bone cement deeply enters a vertebral canal, nerves will be compressed, and the bone cement may enter a blood vessel, which may cause the pulmonary embolism. In some embodiments, the bag of the present disclosure is made of non-braided fiber, and the micro-pores in the bag are formed through post-processing, the distribution of the micro-pores in the bag is controllable, whereas, the holes of the braided fiber are spread across the surface of the bag, and the distribution of the holes is uncontrollable.
5. According to anatomical knowledge, the vertebral body is approximately in an oval shape, and vertebral compression fractures basically occur at the front edge of the vertebral body; in some embodiments, the bag of the present disclosure is in a banana shape or a dumbbell shape, so as to be better conform to the shape of the front edge of the vertebral body, and better maintain the original biomechanical balance of the vertebral body.

### Brief Description of Drawings

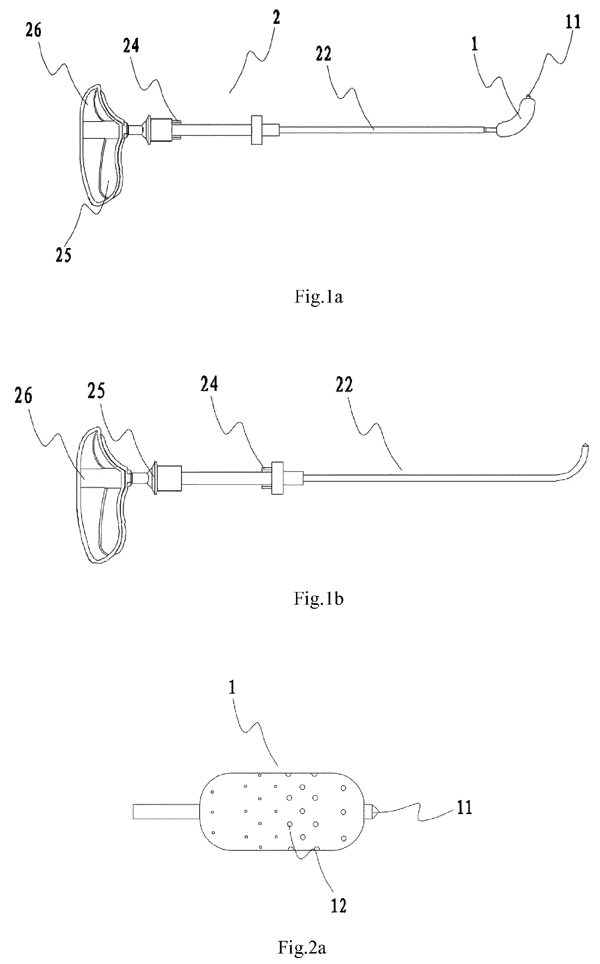

Fig. 1 shows structural schematic diagrams illustrating one embodiment of the present invention, wherein Fig. 1a is a structural schematic diagram when the bag is in a full filling state; Fig. 1b is a structural schematic diagram when the bone filler implanting system is in the initial state.

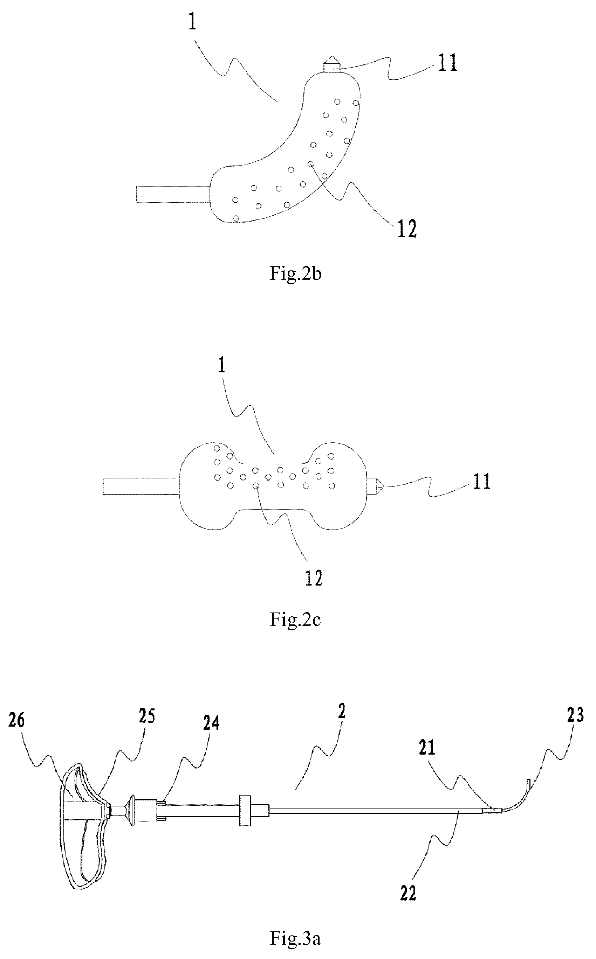

Fig. 2 shows schematic diagrams of the bag according to one embodiment of the present invention, wherein Fig. 2a is a structural schematic diagram of the bag in a straight tubular shape; Fig. 2b is a structural schematic diagram of the bag in a banana shape; and Fig. 2c is a structural schematic diagram of the bag in a dumbbell shape.

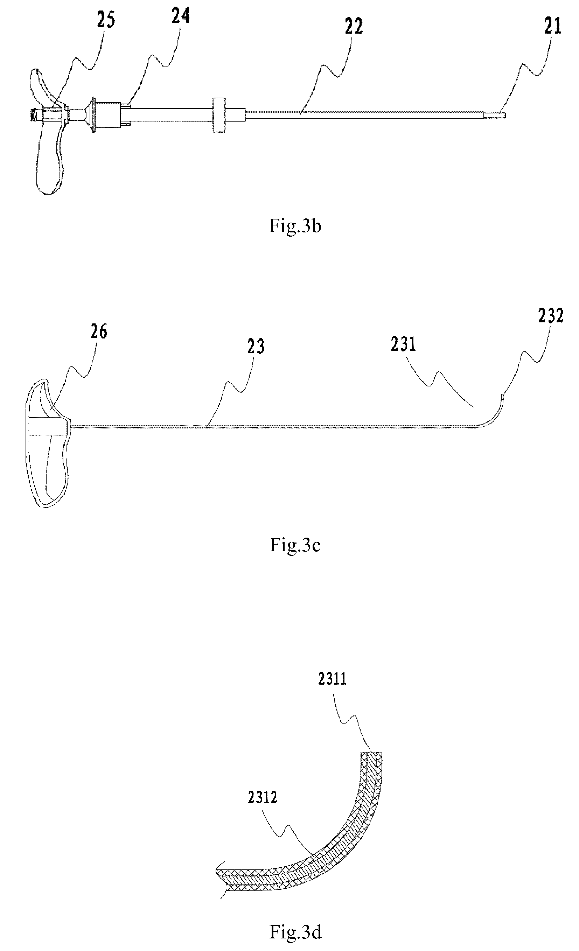

Fig. 3 shows structural schematic diagrams of the delivery device according to one embodiment of the present invention, wherein Fig. 3a is a structural schematic diagram illustrating the delivery device; Fig. 3b is a structural schematic diagram illustrating the delivery tube, the sleeve tube and the delivery handle; Fig. 3c is a structural schematic diagram illustrating the liner core and the liner core handle; and Fig. 3d is a sectional diagram illustrating the curved section of the core tube, which is made of a combination of Nitinol wires and medical polymer material.

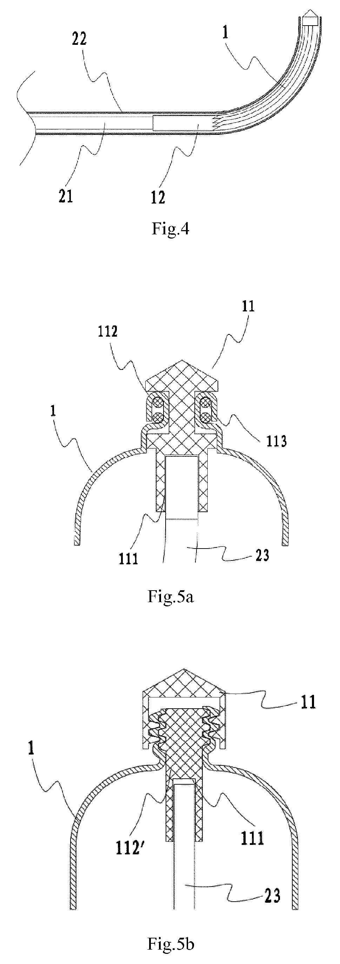

Fig. 4 is a schematic diagram illustrating the relative positions of the sleeve tube, the delivery tube, the liner core and the bag when the bone filler implanting system is in the initial state according to one embodiment of the present invention.

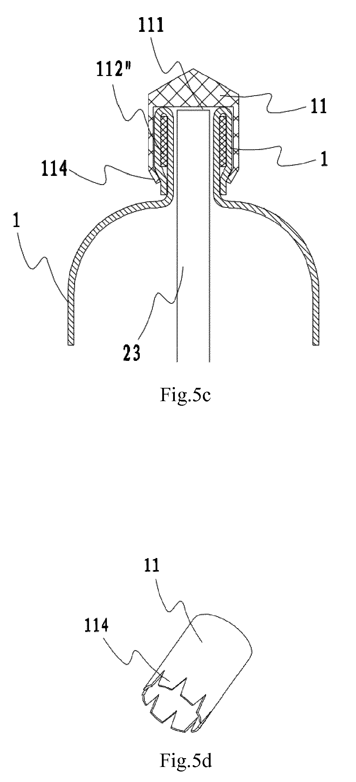

Fig. 5 shows structural schematic diagrams illustrating the sealing head in the present disclosure, wherein Fig. 5a is a structural schematic diagram illustrating the sealing head connected with the bag by means of a binding connection; Fig. 5b is a structural schematic diagram illustrating the sealing head connected with the bag by means of a threaded connection; Fig. 5c is a structural schematic diagram illustrating the sealing head connected with the bag by means of a compression connection; and Fig. 5d is a structural schematic diagram illustrating a compression piece for the compression connection of the sealing head and the bag.

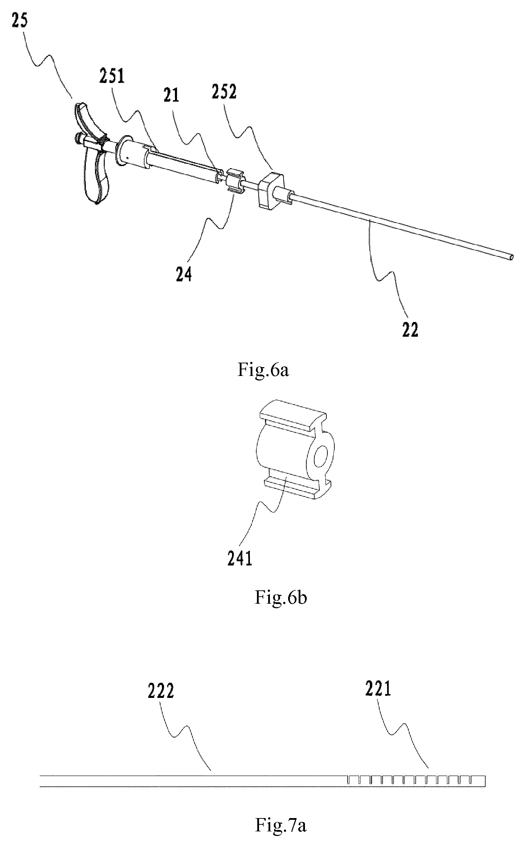

Fig. 6 shows structural schematic diagrams of the delivery handle and the sleeve tube handle according to one embodiment of the present invention, wherein Fig. 6a is a schematic diagram illustrating the assembly relation of the delivery handle and the sleeve tube handle, and Fig. 6b is a structural schematic diagram of a sliding block.

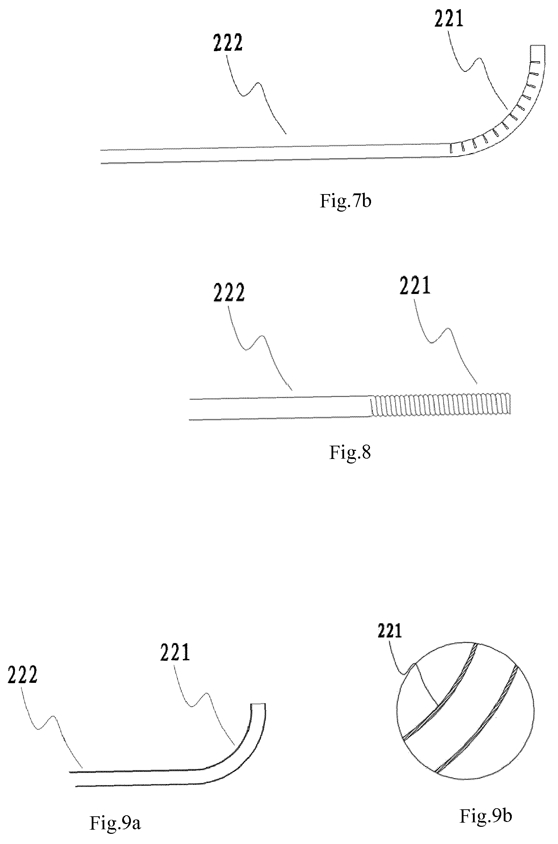

Fig. 7 shows structural schematic diagrams of a first implementation of the sleeve tube according to the present disclosure, wherein Fig. 7a is a schematic diagram illustrating the straight state of the sleeve tube cut from a metal tube, and Fig. 7b is a schematic diagram illustrating the curved state of the sleeve tube cut from a metal tube.
Fig. 8 is a structural schematic diagram of a second implementation of the sleeve tube according to the present disclosure.
Fig. 9 shows local cutaway views of a third implementation of the sleeve tube according to the present disclosure, wherein Fig. 9a is a structural schematic diagram of the sleeve tube made of medical polymer material, and Fig. 9b is a partial enlarged view.

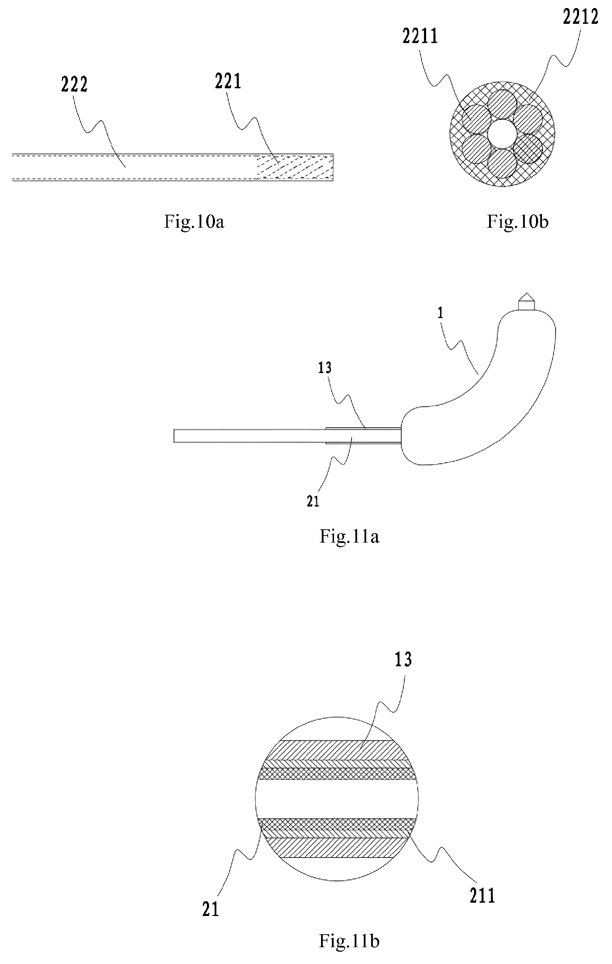

Fig. 10 shows structural schematic diagrams of a fourth implementation of the sleeve tube according to the present disclosure, wherein Fig. 10a is a structural schematic diagram of the sleeve tube made of metal material and medical polymer material, and Fig. 10b is a cross-section view of the sleeve tube.
Fig. 11 shows structural schematic diagrams illustrating a first implementation of the detachable connection between the bag and the delivery tube according to the present disclosure, wherein Fig. 11a is a schematic diagram illustrating the detachable connection in the form of the interference fitting, and Fig. 11b is a partial sectional diagram illustrating the connected section of the bag and the distal end part of the delivery tube.

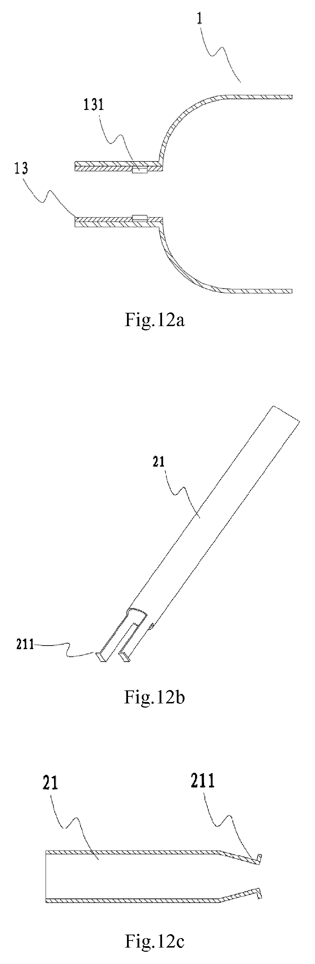

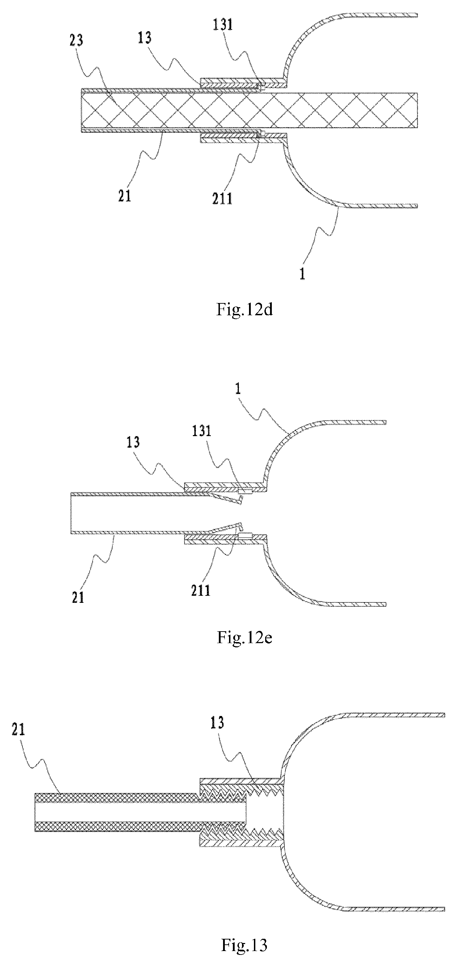

Fig. 12 shows structural schematic diagrams illustrating a second implementation of the detachable connection between the bag and the delivery tube according to the present disclosure, wherein Fig. 12a is a structural schematic diagram illustrating the connection portion of the bag; Fig. 12b and Fig. 12c show the structural schematic diagrams of the snap-fitting joint at the distal end of the delivery tube; Fig.12d is a schematic diagram illustrating the state of the snap-fitting joint when it is fit in position; and Fig.12e is a schematic diagram illustrating the snap-fitting joint in a natural state.
Fig. 13 is a structural schematic diagram illustrating a third implementation of the detachable connection between the bag and the delivery tube according to the present disclosure.

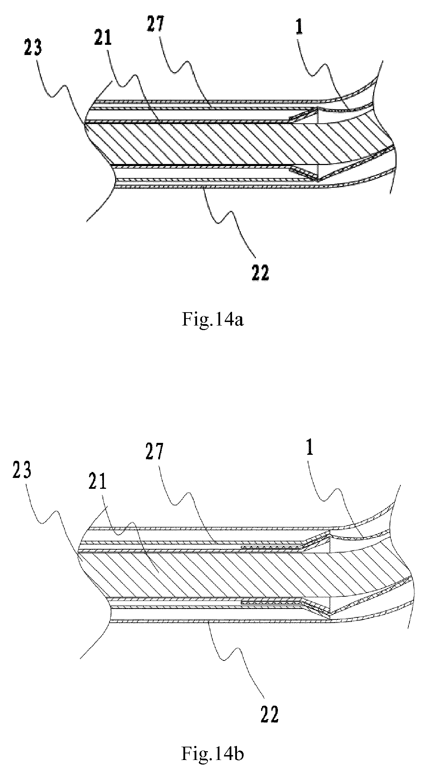

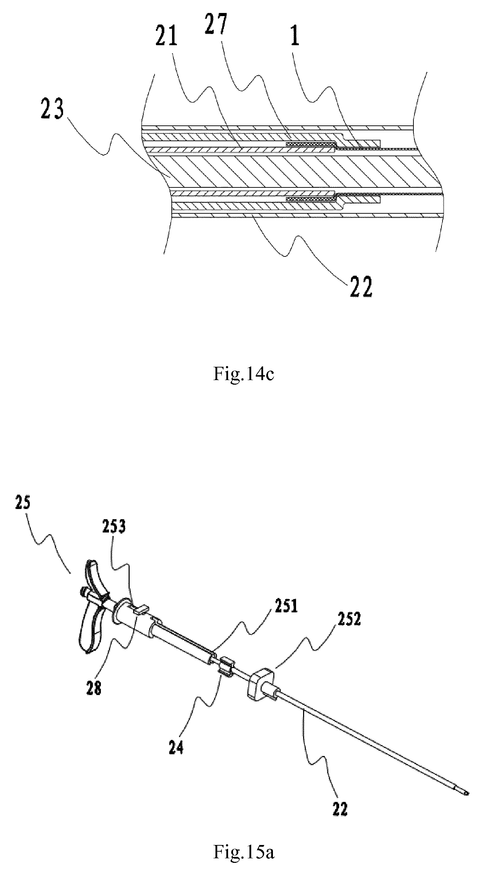

Fig. 14 shows assembly schematic diagrams of the bag, the delivery tube, the pressing tube and the sleeve tube, wherein Fig. 14a is a schematic diagram illustrating the line contact assembly relation of the delivery tube and the pressing tube, wherein the delivery tube has a flared distal end; Fig. 14b is a schematic diagram illustrating the surface contact assembly relation formed by the flared distal end of the delivery tube and the flared distal end of the pressing tube; and Fig. 14c is a schematic diagram illustrating the line contact assembly relation of the delivery tube and the sleeve tube, wherein the pressing tube has a closing distal end.

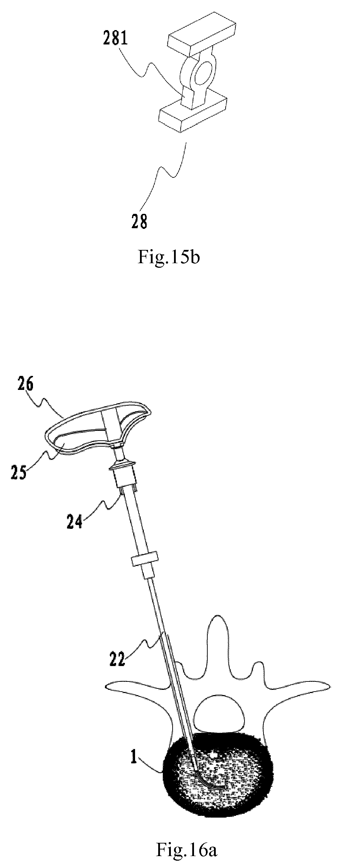

Fig. 15 shows schematic diagrams of the delivery handle, the sleeve tube handle and the pressing tube handle according to one embodiment of the present invention, wherein Fig. 15a is a schematic diagram illustrating the assembly relation of the delivery handle, the sleeve tube handle and the pressing tube handle; and Fig. 15b is a schematic structural diagram of the pressing tube handle.

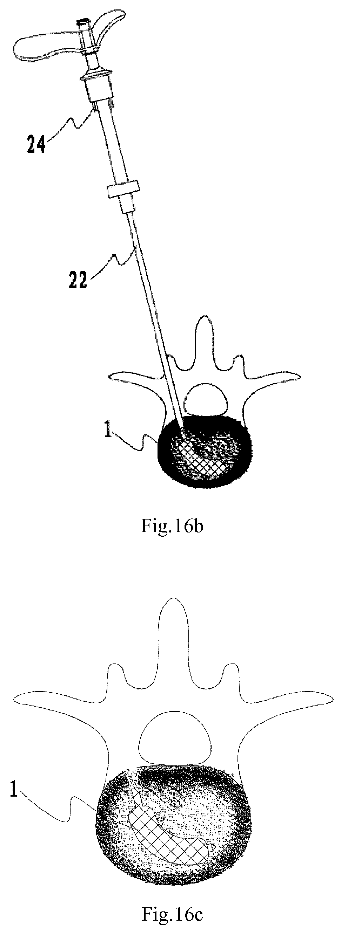

Fig.16 shows schematic diagrams illustrating the bone filler implanting system in service state according to one embodiment of the present invention; wherein, Fig. 16a is a schematic diagram illustrating the service state of the bone filler implanting system after it enters the vertebral body and when the bag is exposed; Fig. 16b is a schematic diagram illustrating the service state of the bone filler implanting system after the bone filler has been injected; and Fig. 16c is a schematic diagram illustrating the service state of the bag after the surgery has been completed.

### Detailed Description of Disclosed Embodiments

In order to make the objectives, technical schemes and advantages of the present disclosure more apparent and better understood, the present disclosure will be described in more details with reference to the accompanying figures and embodiments.

The proximal end as described in the present disclosure refers to the end near to the surgical operator, and the distal end refers to the end far away from the surgical operator.

As shown in Figs. 1-8, according to one embodiment of the present invention, the bone filler implanting system comprises a bag 1 and a delivery device 2. The distal end of the bag 1 is fixedly connected to a sealing head 11, the sealing head 11 is closed at its distal end and has a blind hole 111 at its proximal end, and the blind hole 111 has an opening towards the interior of the bag. The bag 1 has micro-pores 12. The delivery device 2 comprises a delivery tube 21, a sleeve tube 22 and a liner core 23. The delivery tube 21 is rigid, and the distal end of the delivery tube 21 is detachably connected to the proximal end of the bag 1. The liner core 23 is slidably inserted into the delivery tube 21. The distal end part of the liner core 23 is a curved section 231, which extends out of the distal end of the delivery tube 21. The distal end of the liner core 23 is inserted into the blind hole 111 of the sealing head 11 through the delivery tube 21 and the interior of the bag 1. The bag 1 wraps the curved section 231 of the liner core 23. The distal end part of the sleeve tube 22 is a flexible section 221, whereas the proximal end part thereof is a rigid section 222. The flexible section 221 is rigid in the radial direction. The sleeve tube 22 is sleeved on the delivery tube 21 and the bag 1. When the delivery device 2 is operated, the sleeve tube 22 moves axially towards the proximal end of the delivery tube 21, and the bag 1 is exposed.

In one embodiment, as shown in Figs. 2a, 2b and 2c, the bag 1 is in a straight tubular shape; the diameter of the micro-pore disposed at the distal end part of the bag 1 is greater than the diameter of the micro-pore disposed at the proximal end part of the bag 1. The number of the micro-pores disposed in the intermediate part of the bag 1 is larger than the number of the micro-pores disposed at the distal end part and the proximal end part of the bag 1. In another embodiment, as shown in Figs. 2b and 2c, the bag 1 is in a banana shape or a dumbbell shape. The bag 1 has micro-pores 12 which are distributed along circumferential directions on part of the surface of the bag 1. After the bag 1 is connected to the delivery device 2, the part of the bag 1, which are provided with micro-pores 12, faces the arch of the curved section 231 of the liner core 23.

In one of the embodiments, the bag 1 is made of non-braided fiber. For example, it is made of sheet medical polymer material through sintering, or is made of tubular polymer material through blow molding.

As shown in Fig. 5, the distal end of the bag 1 is fixedly connected with a sealing head 11, the distal end of which is closed. The sealing head 11 is made of rigid material. In one embodiment, as shown in Fig. 5a, the proximal end of the sealing head 11 is provided with a blind hole 111, the blind hole 111 has an opening towards the interior of said bag ; the distal end of the sealing head 11 is in a sharp shape; the sealing head 11 is provided with a ring-shaped groove 112; the distal end of the bag 1 is tightly bound to the ring-shaped groove 112 of the sealing head 11 with wires (threads) 113, and the distal end of the bag 1 is turned over and wraps the wires 113. In another embodiment, as shown in Fig. 5b, the sealing head 11 is provided with an inner core 112', the proximal end of which is provided with external threads. The inner core 112' has a blind hole 111, which has an opening towards the interior of the bag 1. The distal end of the sealing head 11 is in a sharp shape and the sealing head 11 is provided with internal threads. The external threads of the inner core 112' and the internal threads of the sealing head 11 are both trapezoidal threads, and the distal end of the bag 1 is tightly clamped between the external threads of the inner core 112' and the internal threads of the sealing head 11, such that the threaded connection is formed. In a third embodiment, as shown in Figs. 5c and 5d, the sealing head 11 is provided with a fixing ring 112", wherein the distal end of the bag 1 passes through the inner hole of the fixing ring 112" and then is turned over outwardly to wrap the fixing ring 112", and the proximal end of the sealing head 11 has several pieces 114 formed by cutting. After the distal end of the bag 1 is turned over to wrap the fixing ring 112", the distal end of the bag is covered with the sealing head 11, and then the pieces 114 provided at the proximal end of the sealing head 11 are pressed down to fixedly connect the distal end of the bag 1 with the sealing head 11, and meanwhile the blind hole 111 is formed inside the fixing ring 112".

In one embodiment, as shown in Fig. 3c, the distal end part of the liner core 23 is the curved section 231 with shape memory property. As shown in Figs. 3c and 3d, the curved section 231 is made of Nitinol wires or a combination of Nitinol wires 2311 and polymer material 2312. The end part of the curved section 231 is a straight section 232, which can be slidably inserted to the blind hole 111 of the sealing head 11 and finally reaches the bottom of the blind hole 111. After the straight section 232 is inserted into the blind hole 111, the sealing head 11 will not be driven when the liner core 23 is withdrawn, thereby avoiding the displacement of the bag 1.

As shown in Fig. 4, the bag 1 is foldable in the axial direction thereof, and wraps the distal end part of the liner core 23 along the circumferential direction of the bag 1.

As shown in Fig. 3 and Fig. 6, the delivery device 2 further comprises a liner core handle 26 fixedly connected to the proximal end of the liner core 23, a delivery handle 25 fixedly connected to the proximal end of the delivery tube 21, and a sleeve tube handle 24 connected to the proximal end of the sleeve tube 22. The delivery handle 25 is provided with one or more guide grooves 251. The distal end of the delivery handle 25 is provided with a stop block 252 for limiting the position of the sleeve tube handle 24, and the sleeve tube handle 24 is provided with one or more guide blocks 241 engaged with the guide grooves 251 of the delivery handle 25, such that, when the sleeve tube handle 24 is moved, the sleeve tube 22 moves axially together with the sleeve tube handle 24.

As shown in Figs.7-10, the distal end part of the sleeve tube 22 is a flexible section 221, whereas the proximal end part thereof is a rigid section 222, wherein the flexible section 221 is rigid in the radial direction; the sleeve tube 22 is sleeved on the delivery tube 21 and the bag 1; and the distal end of the sleeve tube 22 is sleeved on or connected to the sealing head 11. When the delivery device 2 is operated, the sleeve tube 22 moves axially to the proximal end of the delivery tube 21, and the bag 1 is exposed. The flexible section of the sleeve tube 22 is cut from a metal tube, formed of a spring, or made of hard medical polymer material or a combination of metal material or polymer material. In one embodiment, as shown in Figs. 7a and 7b, the flexible section 221 of the sleeve tube 22 is cut from a metal tube, and the rigid section 222 is a metal tube. In another embodiment, as shown in Fig. 8, the flexible section 221 of the sleeve tube 22 is formed of a metal spring, and the rigid section 222 is a metal tube, wherein the proximal end of the flexible section 221 is welded to the distal end of the rigid section 222. In a third embodiment, as shown in Figs. 9a and 9b, the flexible section 221 of the sleeve tube 22 is formed by a curved section made of medical polymer material (preferably, PEEK) through heat-setting. In a fourth embodiment, as shown in Figs. 10a and 10b, the flexible section 221 of the sleeve tube 22 is made of a combination of metal material 2211 and medical polymer material 2212, and the rigid section 222 is made of metal material.

As shown in Figs. 11 to 13, the distal end of the delivery tube is detachably connected with the proximal end of the bag. The detachable connection includes an interference fit connection, a snap-fitting joint connection or a threaded connection. In one embodiment, as shown in Figs. 11a and 11b, the proximal end of the bag 1 extends to form a tubular section 13. Outer surface of the distal end of the delivery tube 21 is covered with a layer of elastic film 211 with a larger friction coefficient. The tubular section 13 is directly sleeved on the distal end of the delivery tube 21 to form an interference fit. In another embodiment, as shown in Fig. 12a, the proximal end of the bag 1 is provided with a hard tube 13, and the hard tube 13 has one or more holes 131. The distal end of the bag 1 is fixed on the outer surface of the hard tube 13 through heating and pressing. The holes 131 are covered but not filled by the proximal end part of the bag 1. As shown in Figs. 12b, 12c, 12d and 13e, the distal end of the delivery tube 21 has one or more snap joints 211. The snap joints 211 are formed by cutting and shaping the distal end of the delivery tube 21, and the snap joints 211 are bent in the natural state. When the liner core 23 is inserted into the delivery tube 21 and goes through the snap joints 211, the snap joints 211 are propped up and snapped into the holes 122 of the bag 1. When the liner core 23 is withdrawn, the snap joints 211 are restored to the natural state and are released from the holes 122. In a third embodiment, as shown in Fig. 13, the proximal end of the bag 1 is provided with a hard tube 13, and the hard tube 13 has internal threads. The proximal end of the bag 1 is fixed on the outer surface of the hard tube 13 through heating and pressing. The distal end of the delivery tube 21 has external threads which are engageable with the internal threads of the hard tube 13.

As shown in Figs. 14 and 15, a pressing tube 27 is arranged between the delivery tube 21 and the sleeve tube 22 of the bone filler implanting system, namely, the pressing tube 27 is sleeved on the delivery tube 21, and the sleeve tube 22 is sleeved on the pressing tube 27. The proximal end of the pressing tube 27 is fixedly connected with the pressing tube handle 28. The distal end of the delivery tube 21 or the distal end of the pressing tube 27 is subject to deforming treatment, so that a line contact or a surface contact is formed when the distal end of the delivery tube 21 and the distal end of the pressing tube 27 are assembled coaxially, thereby the proximal end of the bag 1 is fixed between the pressing tube 27 and the delivery tube 21. In one embodiment, as shown in Fig. 14, the distal end of the delivery tube 21 is formed into a bell mouth, or is fixed attached with an annular bulge, or is flared into a stepped structure, so that a line contact is formed between the pressing tube 27 and the distal end of the delivery tube 21, and the proximal end of the bag 1 is fixed between the pressing tube 27 and the delivery tube 21. The pressing tube 27 can be moved axially by operating the pressing tube handle 28, thereby releasing the bag 1. In another embodiment, the distal end of the pressing tube 27 is formed into a flared opening or a stepped structure, so that a surface contact is formed between the distal end of the pressing tube 27 and the distal end of the delivery tube 21, and the proximal end of the bag 1 is fixed between the delivery tube 21 and the pressing tube 27. In a third embodiment, the distal end of the pressing tube 27 is formed into a closing opening, so that a line contact is formed between the closing opening of the pressing tube 27 and the distal end of the delivery tube 21, and the proximal end of the bag 1 is fixed between the pressing tube 27 and the delivery tube 21. The pressing tube 27 can be moved axially by operating the pressing tube handle 28, thereby releasing the bag 1. In a fourth embodiment, the distal end of the delivery tube 21 is formed into a closing opening or a step, so that a surface contact is formed between the delivery tube 21 and the distal end of the pressing tube 27, and the proximal end of the bag 1 is fixed between the pressing tube 27 and the delivery tube 21.

In one embodiment, as shown in Fig. 15, the pressing tube handle 28 is installed on the delivery handle 25. A sliding chute 253 is provided in the delivery handle 25, and a raised stop block 281 is arranged on the pressing tube handle 28. When the pressing tube handle 28 is installed on the delivery handle 25, the stop block 281 gets stuck in the sliding chute 253. The pressing tube 27 can be moved axially by moving the pressing tube handle 28.

In the above-mentioned embodiments, the sealing head 11, the inner core 112' and the fixing ring 112" are made of metal material (preferably, pure titanium) or hard medical polymer material (preferably, PEEK). The bag 1 is made of medical polymer material (preferably, expanded PTFE). The delivery tube 21 and the pressing tube 27 are formed of metal tubes (preferably, 304 stainless steel), the flexible section 221 of the sleeve tube 22 is cut from a metal tube, formed of a spring or shaped from medical polymer material (preferably, PEEK); and the rigid section of the sleeve tube 22 is formed of a metal tube or made of medical polymer material (preferably, PEEK). The film 211 at the distal end of the delivery tube 21 is made of medical polymer material (preferably, expanded PTFE). The liner core 23 is made of shape memory alloy (preferably, Nitinol). The sleeve tube handle 24, the delivery handle 25, the liner core handle 26 and the pressing tube handle 28 are made of medical polymer material (preferably, POM).

In surgical procedures, after a working channel is established, the bone filler implanting system is inserted into the vertebral body through the working channel up to a predetermined position under the observation with a C-arm, and during the procedure, the liner core 23 has the following functions:
1. The distal end of the liner core 23 abuts against the blind hole 111 of the sealing head 11, such that the bag 1 can be prevented from moving or turning over in the sleeve tube 22 during the delivery procedure, and the sealing head 11 can be driven to push away the bone tissue by pushing the liner core 23.
2. The bag 1 is configured to wrap the curved section 231 of the liner core 23, such that the bag can be fed to the center of the vertebral body under the protection of the sleeve tube 22.

As shown in Figs. 16a, 16b and 16c, after the bone filler implanting system is inserted to the predetermined position, the sleeve tube handle 24 is moved towards the proximal end to make the sleeve tube 22 move backwards slowly, till the bag 1 is exposed entirely; at this time, the liner core handle 26 is pulled to withdraw the liner core 23 completely, and after that, the bone filler implanting system is coupled to the delivery device handle 21 to inject the bone filler into the bag 1 through the delivery channel, till the bag 1 is dilated. When the bone filler begins to cure, pull the delivery device handle 21 to make the bag 1 released from the delivery tube 21. Finally, withdraw the delivery tube 21. As for the embodiment that the distal end of the delivery tube 21 is connected with the proximal end of the bag 1 through threads, when the bone filler begins to cure, the delivery device handle 21 needs to be rotated to make the bag 1 released from the threads of the delivery tube 21; and finally, withdraw the delivery tube 21.

In the embodiment that the pressing tube is arranged, after the bone filler is injected into the bag 1 through the delivery channel, slide the pressing tube handle 28 axially to drive the pressing tube 27 to move, thereby releasing the bag 1, and finally, withdraw the delivery device 2.

Finally, it should be noted that the above mentioned are only preferred embodiments of the present invention, but not to limit the scope of the invention, and any amendments, equivalent replacements, improvements and so on made within the spirits and principles of the present invention all should be included in the protection scope of the present invention.

## Claims

1. A bone filler implanting system, comprising a bag and a delivery device, wherein, a distal end of the bag is fixedly connected to a sealing head; the sealing head is closed at its distal end and has a blind hole at its proximal end, and the blind hole has an opening towards an interior of the bag; the bag has micro-pores; the delivery device comprises a liner core, a delivery tube and a sleeve tube; the delivery tube is rigid, and a distal end of the delivery tube is detachably connected to a proximal end of the bag; the liner core is slidably inserted into the delivery tube; a distal end part of the liner core is a curved section, which extends out of the distal end of the delivery tube; a distal end of the liner core is configured to be inserted into the blind hole of the sealing head through the delivery tube and the interior of said bag; the bag is configured to wrap the curved section of the liner core; a distal end part of the sleeve tube is a flexible section, whereas a proximal end part of the sleeve tube is a rigid section, the flexible section is rigid in its radial direction; the sleeve tube is sleeved on the delivery tube and the bag; when the delivery device is operated, the sleeve tube moves axially towards a proximal end of the delivery tube, and the bag is exposed.

2. The bone filler implanting system according to claim 1, wherein, a diameter of the micro-pore disposed at the proximal end part of the bag is less than the diameter of the micro-pore disposed at the distal end part of the bag; or the number of the micro-pores disposed in an intermediate part of the bag is larger than the number of the micro-pores disposed at two end parts of the bag; or the micro-pores are provided at one flank of the bag; or any combination of above.

3. The bone filler implanting system according to claim 1, wherein, the bag is in a banana shape or a dumbbell shape.

4. The bone filler implanting system according to claim 1, wherein, the bag is made of non-braided fiber.

5. The bone filler implanting system according to claim 1, wherein, the bag is foldable in its axial direction, and is configured to wrap the distal end part of the liner core along a circumferential direction of the bag.

6. The bone filler implanting system according to claim 1, wherein, an end part of the curved section is a straight section, which can be slidably inserted to the blind hole of the sealing head and finally reach the bottom of the blind hole.

7. The bone filler implanting system according to claim 1, wherein, the delivery device further comprises a liner core handle fixedly connected to a proximal end of the liner core, a delivery handle fixedly connected to the proximal end of the delivery tube, and a sleeve tube handle connected to a proximal end of the sleeve tube; the delivery handle is provided with one or more guide grooves; a distal end of the delivery handle is provided with a stop block for limiting a position of the sleeve tube handle; and the sleeve tube handle is provided with one or more guide blocks engaged with the guide grooves, such that, when the sleeve tube handle is moved, the sleeve tube moves axially together with the sleeve tube handle.

8. The bone filler implanting system according to claim 1, wherein, the flexible section of the sleeve tube is cut from a metal tube, is formed of a spring, or is made of a hard medical polymer material or a combination of metal material or polymer material.

9. The bone filler implanting system according to claim 1, wherein, a pressing tube is arranged between the delivery tube and the sleeve tube; a distal end of the pressing tube is fixedly connected with a pressing tube handle; the distal end of the delivery tube or the distal end of the pressing tube is subject to deforming treatment, so that a line contact or a surface contact is formed when the distal end of the delivery tube and the distal end of the pressing tube are assembled coaxially, thereby the proximal end of the bag is fixed between the pressing tube and the delivery tube.

10. The bone filler implanting system according to claim 9, wherein, the deforming treatment is flaring, closing, forming a step, fixing a bulge, or enlarging an inner cavity.

11. The bone filler implanting system according to claim 1, wherein: the sealing head is made of rigid material; a distal end of the sealing head is in a sharp shape; and the distal end of the sleeve tube is sleeved on or connected to the sealing head.
